# EUROPEAN PATENT APPLICATION

(11) **EP 4 230 651 A1**
(43) Date of publication of application: **23.08.2023**
(21) Application number: 21880498.7
(22) Date of filing: 13.10.2021
(51) Int. Cl.: C07K 16/26, C07K 14/47, C12N 15/62, A61P 17/14, A61K 8/64, A61Q 7/00, A61K 39/00

(54) **TESTOSTERONE-SPECIFIC AFFIBODY AND USES THEREOF**

(30) Priority: 13.10.2020 KR 20200132203
(71) Applicant: Inssen Inc., Seoul 08381 (KR)
(72) Inventor: PARK, Hye-Sun, Seongnam-si Gyeonggi-do 13473 (KR); CHO, Hyun-Nam, Siheung-si Gyeonggi-do 14994 (KR); JANG, Dai Ho, Seoul 03747 (KR)
(74) Representative: J A Kemp LLP
(86) International application number: PCT/KR2021/014103
(87) International publication number: WO 2022/080851

(57) **Abstract**

The present invention relates to an affibody that binds to testosterone, and uses thereof. An affibody or a complex of affibody and osteopontin fragments, of the present invention, has superior effects on hair growth or hair loss prevention, and thus can be effectively used as a hair growth agent or a hair loss treatment agent.

## Description

### Technical field

This patent application claims priority over Korean Patent Application No. 10-2020-0132203 filed in the Korean Intellectual Property Office on October 13, 2020, and the disclosure of which is incorporated herein by reference.

The present disclosure relates to a testosterone-specific affibody and a use thereof.

### Background art

Hair loss refers to a disease in which hair or body hair is lost due to various causes such as genetic factors, aging, stress, nutritional imbalance, and male hormones. Types of hair loss include androgenic alopecia, which is chronic hair loss, traumatic hair loss, and chemical hair loss. There are two types of hair loss treatments licensed by the US Food and Drug Administration (FDA) to date: Minoxidil and Finasteride. Although the hair growth mechanism of Minoxidil has not yet been precisely identified, it is known to be related to the effect of promoting nutrient supply to dermal papilla cells by increasing the expression of vascular endothelial growth factor (VEGF).

Finasteride is a substance that inhibits the production of dihydrotestosterone (DHT), which causes male hair loss and prostatic hyperplasia, and blocks the function of 5-alpha-reductase. However, both Minoxidil, which is a topic agent, and Finasteride, which is an oral therapeutic agent, cannot be used for women of childbearing age and pregnant women. In particular, Finasteride, when taken, it systemically inhibits the conversion of testosterone to DHT, resulting significant side effects such as a decrease in sexual function. In addition, in order to actually treat hair loss, both Minoxidil, which increases vascular endothelial growth factor around dermal papilla cells, and Finasteride, which blocks 5-alpha-reductase, are prescribed to hair loss patients, so that the patients have to separately administer two kinds of drugs, causing inconvenience in use. Accordingly, there is a growing demand for the development of new hair loss treatments that can improve the inconvenience of drug administration while having fewer systemic side effects.

Numerous papers and patent literatures are referenced throughout the present specification and citations thereof are indicated. The disclosures of the cited papers and patent literatures are incorporated herein by reference in their entirety, so as to more clearly describe the level of the technology to which the present disclosure pertains and the contents of the present disclosure.

### Detailed description of the invention

### Technical problem

The present inventors have made intensive research efforts to develop a novel hair loss treatment agent having excellent effects on treating hair loss by reducing side effects and inconvenience of using the conventional hair loss treatment agents.

As a result, the inventors have developed a novel polypeptide (affibody) that binds to free testosterone and a novel complex in which the polypeptide that binds to testosterone is combined with a angiogenic polypeptide, and found that their effect of stimulating hair growth or treating hair loss is excellent.

Accordingly, it is an object of the present disclosure to provide a polypeptide that specifically binds to testosterone.

It is another object of the present disclosure to provide a complex comprising the polypeptide and at least one angiogenic polypeptide.

It is another object of the present disclosure to provide a pharmaceutical composition for stimulating hair growth or treating hair loss comprising the polypeptide or the complex as an active ingredient.

It is yet another object of the present disclosure is to provide a cosmetic composition for stimulating hair growth, comprising the polypeptide or the complex as an active ingredient.

### Technical Solution

As used herein, the term "affibody" molecule is a Z-domain, which is a site having affinity for IgG among protein A of *Staphylococcus aureus,* and is a small protein consisting of 58 amino acid residues. The 14 amino acids forming the IgG-binding surface in the protein sequence of these affibody molecules can bind to various target antigens according to the amino acid sequence, and can be randomly arranged to build a library. Affibody molecules that can bind to various target antigens may be selected from libraries similar to antibodies through screening methods such as phage display, yeast two hybrid (Y2H), and the like. An affibody specifically binding to HER2 and amyloid-beta has been recently developed using the properties of an affibody molecule capable of binding to a target antigen (Orlova et al. 2006, Cancer Res., Gronwall et al., 2007, J. Biotechnol.). In addition, affibody has a very small molecular weight of 6 kDa, and thus has a feature that is systemically diffused when administered to a human body and is rapidly removed by renal filtration, compared to an antibody in an IgG form having a molecular weight of 150 kDa. Therefore, affibody is mainly applied to research and development of diagnostic samples (Goldstein R et al., 2013, Expert Rev Anticancer Ther.). Affibody has also been developed in the form of a bispecific antibody bound to general IgG (Yu F et al., 2014, MAbs).

The invention related to polypeptide scaffolds based on the first-generation Z variants has been disclosed in PCT Publication WO95/19374, and the invention related to polypeptide scaffolds based on second generation Z variants has been disclosed in PCT Publication WO2009/080811.

According to an aspect thereof, the present disclosure provides a polypeptide that specifically binds to testosterone, comprising an amino acid sequence having at least 94% sequence homology with the following amino acid sequence of SEQ ID NO: 1:

VDNKFNKEMVQAMREISYLPNLNHTQIRAFIWVLFDDPSQSANLLAEAKKLNDAQA
PK (SEQ ID NO: 1).

The polypeptide comprises the affibody^{®} molecule described above.

As used herein, the term "affinity" refers to a property or ability to specifically bind to a specific subject. In the biological field such as the present disclosure, the term is used to indicate the strength of binding force between specific substances, for example, the ability of an enzyme and a substrate to bind to each other, the ability of an antibody to bind to an antigen, and the like.

As used herein, the term "amino acid" refers to the most basic feature unit of a protein molecule. In the structure of the amino acid, an amino group (-NH₂) and a carboxyl group (-COOH) are attached to one carbon atom, to which a hydrogen and R group are connected.

As used herein, the term "protein" refers to a polymer organic material that constitutes the body of all living organisms and is a polymer of numerous amino acids. There are about 20 kinds of natural amino acids, and the amino acids are connected to each other by a chemical bond called a peptide bond to form a long side chain, which is called a polypeptide. The polypeptide of the present disclosure may be prepared by a synthesis method known in the art, for example, by transforming a host cell with an expression vector containing a nucleic acid molecule encoding a protein to synthesize a recombinant protein or by solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111 (1984)).

The polypeptide of the present disclosure that specifically binds to testosterone may comprise a variant of an amino acid sequence within a range capable of specifically recognizing testosterone, as recognized by those skilled in the art. For example, variation can be added to the amino acid sequence of the polypeptide may be to improve the binding affinity and/or other biological properties of the polypeptide that specifically binds to testosterone. Such modifications include, for example, deletion, insertion and/or substitution of the amino acid residues of the polypeptide.

The variant has "substantial similarity" which means that the two peptide sequences, when optimally aligned, such as by the programs GAP or BESTFIT using default gap weights, share at least about 90% sequence identity, more preferably at least about 91%, 92%, 93%, 94%, 95%, 96%, 97%, 98% or 99% sequence identity. Preferably, residue positions that are not identical differ by conservative amino acid substitutions. A "conservative amino acid substitution" refers to a substitution of an amino acid residue by another amino acid residue having a side chain (R group) with similar chemical properties (e.g., charge or hydrophobicity). In general, conservative amino acid substitutions do not substantially change the functionality of the protein.

Such amino acid mutations are made based on the relative similarity of amino acid side chain substituents, such as hydrophobicity, hydrophilicity, charge, size, and the like. By the analysis of the size, shape and type of amino acid side chain substituents, it can be seen that arginine, lysine and histidine are all positively charged residues; alanine, glycine and serine have similar sizes; and phenylalanine, tryptophan and tyrosine have similar shapes. Therefore, based on these considerations, arginine, lysine and histidine; alanine, glycine and serine; and phenylalanine, tryptophan and tyrosine can be said to be biologically functional equivalents.

When introducing mutations, the hydropathic index of amino acids can be considered. Each amino acid has been assigned a hydrophobic index according to its hydrophobicity and charge: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The hydropathic amino acid index is very important in imparting an interactive biological function of a protein. It is a known fact that similar biological activity can be retained only by substitution with an amino acid having a similar hydropathic index. When introducing a mutation with reference to the hydropathic index, the substitution is performed between amino acids showing a difference in the hydropathic index, preferably within ±2, more preferably within ±1, and even more preferably within ±0.5.

Meanwhile, it is also well known that substitution between amino acids having similar hydrophilicity values results in proteins having equivalent biological activity. As disclosed in U.S. Patent No. 4,554,101, the following hydrophilicity values are assigned to each amino acid residue: arginine (+3.0); lysine (+3.0); aspartate (+3.0±1); glutamate (+3.0±1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

When the mutation is introduced with reference to the hydrophilicity value, the substitution is performed between amino acids exhibiting a difference in the hydrophilicity value within preferably ±2, more preferably within ±1, and even more preferably within ±0.5.

Amino acid exchanges in proteins that do not generally alter the activity of the molecule are known in the state of the art (H. Neurath, R.L.Hill, The Proteins, Academic Press, New York, 1979). The most commonly occurring exchanges are those between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly.

In one embodiment of the present disclosure, the polypeptide of the present disclosure that specifically binds to testosterone comprises an amino acid sequence having at least 94% sequence homology (e.g., 94%, 95%, 96%, 97, 98%, or 99%) with the amino acid sequence of SEQ ID NO: 1. All integers of 94% or more and 100% or less and prime numbers therebetween are included within the scope of the present disclosure with respect to % homology.

In one embodiment of the present disclosure, the polypeptide of the present disclosure that specifically binds to testosterone is a polypeptide wherein at least one amino acid residue selected from glutamine residue (Q) at position 11, serine residue (S) at position 17, and threonine residue (T) at position 25 of the following amino acid sequence is independently replaced with any amino acid residue:

VDNKFNKEMVQAMREISYLPNLNHTQIRAFIWVLFDDPSQSANLLAEAKKLNDAQA
PK.

In another embodiment of the present disclosure, the polypeptide of the present disclosure that specifically binds to testosterone comprises an amino acid sequence having at least 98% sequence homology with the amino acid sequence of SEQ ID NO: 1.

In a specific embodiment of the present disclosure, the polypeptide of the present disclosure that specifically binds to testosterone comprises an amino acid sequence wherein the glutamine residue (Q) at position 11, the serine residue (S) at position 17, or the threonine residue (T) at position 25 of the following amino acid sequence is replaced with alanine residue (A):

VDNKFNKEMVQAMREISYLPNLNHTQIRAFIWVLFDDPSQSANLLAEAKKLNDAQA
PK.

In another specific embodiment of the present disclosure, the polypeptide of the present disclosure that specifically binds to testosterone comprises an amino acid sequence selected from the group consisting of amino acid sequences of SEQ ID NOs: 5 to 7, 9 to 14, 16, and 20 to 22.

In another specific embodiment of the present disclosure, the polypeptide specifically binding to testosterone of the present disclosure comprises all of the underlined amino acid residues in the following amino acid sequence:

VDNKFNKEMVQAMREISYLPNLNHTQIRAFIWVLFDDPSQSANLLAEAKKLNDAQA
PK.

Amino acid residues underlined above indicate amino acid residues at positions that specifically binds to a target. A polypeptide that binds to a target, also known as an affibody comprises an amino acid sequence of 58 amino acid residues. The amino acid residues at the positions serve as a complementarity-determining region (CDR) of an antibody. An affibody may be prepared as a polypeptide that binds to various targets by altering the amino acid residues at those positions among 58 amino acid residues. The amino acid residues other than the underlined amino acid residues serve to maintain the triple helix polypeptide structures in the affibody. Accordingly, in an embodiment of the present disclosure, the polypeptide of the present disclosure that specifically binds to testosterone comprises an affibody molecule comprising another different scaffold sequence having the same type of amino acid residues at the underlined position.

According to another aspect thereof, the present disclosure provides a complex comprising a polypeptide that specifically binds to the testosterone and at least one angiogenic polypeptide.

In this case, the complex is in the form of a multimer in which each polypeptide monomer is linked. In the complex of the present disclosure, each polypeptide is covalently linked to each other, and according to an embodiment of the present disclosure, the complex may be implemented in the form of a fused protein or a conjugate.

In an embodiment of the present disclosure, the angiogenic polypeptide comprises osteopontin or a fragment thereof.

In a specific embodiment of the present disclosure, the fragment of osteopontin comprises the amino acid sequence (SVYGLR) of SEQ ID NO: 66. The amino acid sequence (SVYGLR) of SEQ ID NO: 66 is a cleaved epitope of osteopontin that is cleaved by thrombin.

In another specific embodiment of the present disclosure, the fragment of osteopontin may comprise SVVYGLR (OPN-R) which is an epitope cleaved by the thrombin, for example, GRGDSVVYGLRS, GRGDSVVYGLR, RGDSVVYGLR, SVVYGLRS, or RGDSVVYGLR. In another specific embodiment of the present disclosure, the fragment of osteopontin may be OPN-L (SWYGL) formed by further cleavage of arginine at the C-terminus by carboxypeptidase B (CPB).

The fragments of osteopontin and amino acid sequences thereof described above have been used as one of the examples of angiogenesis promoting polypeptides, and thus should not be construed as limiting the scope of the present disclosure. Therefore, the angiogenic polypeptide included in the complex of the present disclosure may be any other polypeptide having a suitable angiogenic function.

In one embodiment of the present disclosure, the angiogenic polypeptide (e.g., a fragment of osteopontin) of the present disclosure may be fused to the N-terminus and/or the C-terminus, more specifically to the N-terminus or the C-terminus, and most specifically to the C-terminus of the polypeptide of the present disclosure that specifically binds to testosterone (i.e., an affibody molecule), but is not limited thereto. However, it is preferable in terms of an angiogenic effect that the angiogenic polypeptide is fused to the C-terminus of the polypeptide that specifically binds to testosterone.

In an embodiment of the present disclosure, in the complex, the polypeptide that specifically binds to testosterone and the angiogenic polypeptide may be directly linked or indirectly linked via a linker (e.g., an amino acid linker).

For those skilled in the art will appreciate that when preparing a fusion protein, it may usually involve the use of linkers between functional moieties to be fused, and that there are different types of linkers with different properties, such as flexible amino acid linkers, non-flexible linkers and cleavable amino acid linkers. The linker has been used to increase the expression level of the fusion protein, to improve biological activity, to enable targeting, to change pharmacokinetics, or to increase the stability of the fusion protein and to improve folding.

Thus, according to a specific embodiment of the present disclosure, the complex may further comprise at least one linker, for example, at least one linker selected from a flexible amino acid linker, a non-flexible linker, and a cleavable amino acid linker. According to the most specific embodiment of the present disclosure, the linker is arranged between the affibody molecule and osteopontin or a fragment thereof.

In another embodiment, the complex may comprise at least one additional amino acid at the C-terminus and/or the N-terminus. The additional amino acid residues may be added individually or collectively for the purpose of improving, for example, productivity, purification, *in vivo* or *ex vivo* stabilization, coupling or detection of complexes. For example, a cysteine residue may be added to the C-terminus and/or the N-terminus of the complex. Additional amino acid residues may also provide a "tag" for the detection of a tablet or polypeptide, for example, a tag for interaction with an antibody specific for the tag, a tag such as an His6 tag, a (HisGlu)3 tag ("HEHEHE" tag) for immobilized metal affinity chromatography (IMAC) of an His6 tag, a "myc" (c-myc) tag or a "FLAG" tag.

Additional amino acids as described above may be linked by chemical conjugation to i) a polypeptide that binds to testosterone, or ii) a complex comprising a polypeptide that binds to testosterone and an angiogenic polypeptide as defined herein, or may be expressed as a fusion protein, and may be linked directly or indirectly via a linker (e.g., an amino acid linker).

In an embodiment of the present disclosure, i) the testosterone-binding polypeptide and ii) the angiogenesis promoting polypeptide of the complex are linked by at least one linker.

In this case, the linker may consist of an amino acid sequence represented by the general formula (GnSm)p or (SmGn)p:
wherein each of n, m and p are independent;
n is an integer from 1 to 7;
m is an integer from 0 to 7;
the sum of n and m is an integer of 8 or less; and
p is an integer from 1 to 7.

In another embodiment of the present disclosure, n of the linker is an integer from 1 to 5, and m is an integer from 0 to 5. For another specific embodiment, the linker is GGGGS. For another specific embodiment, the linker is GGGG.

In another embodiment of the present disclosure, the linker may be VDGS or ASGS, but is not limited thereto.

In another aspect thereof, the present disclosure provides a nucleic acid molecule comprising a nucleotide sequence encoding i) a polypeptide that binds to testosterone, or ii) a complex comprising the polypeptide that binds to testosterone and an angiogenic polypeptide, as described above.

As used herein, the term "nucleic acids" is meant to include DNA (gDNA and cDNA) and RNA molecules inclusively, and nucleotides, which are the basic building blocks of a nucleic acid molecule, includes not only naturally occurring nucleotides but also analogues modified at the sugar or base positions (Scheit, Nucleotide Analogs, John Wiley, New York(1980); Uhlman an d Peyman, Chemical Reviews, 90:543-584(1990)).

In one embodiment of the present disclosure, it is apparent to those in the art that the nucleotide sequence encoding the polypeptide or the complex of the present disclosure is sufficient if it is a nucleotide sequence encoding the polypeptide or an amino acid sequence constituting the complex, and is not limited to any specific nucleotide sequence.

This is because even if a nucleotide sequence mutation occurs, expression of the mutated nucleotide sequence as a protein may result in no change in the protein sequence. This is called codon degeneracy. Therefore, the nucleotide sequence includes nucleotide sequences that contain functionally equivalent codons or codons encoding the same amino acids (e.g., due to codon degeneracy, arginine or serine has six codons), or codons encoding biologically equivalent amino acids.

Considering the mutation having the above-mentioned biological equivalent activity, nucleic acid molecules encoding the polypeptides or complexes of the present disclosure are construed as including sequences exhibiting substantial identity to the sequences set forth in the sequence listing. When the sequence of the present disclosure and any other sequences are aligned so as to correspond to each other as much as possible, and the aligned sequence is analyzed using an algorithm commonly used in the art, the substantial identity means sequences exhibiting at least 60% homology (e.g., 60%, 61%, 62%, 63%, 64%, 65%, 66%, 67%, 68%, or 69%), more preferably at least 70% homology (e.g., 70%, 71%, 72%, 73%, 74%, 75%, 76%, 77%, 78%, or 79%), even more preferably at least 80% homology (e.g., 80%, 81%, 82%, 83%, 84%, 85%, 86%, 87%, 88%, or 89%), most preferably at least 90% homology (e.g., 90%, 91%, 92%, 93%, 94%, 95%, 96%, 97%, 98%, 99%, or 100%). All integers of 70% or more and 100% or less and prime numbers therebetween are included within the scope of the present disclosure with respect to % homology.

Alignment methods for sequence comparison are known in the art. Various methods and algorithms for alignment are disclosed in Smith and Waterman, Adv. Appl. Math. 2:482(1981); Needleman and Wunsch, J. Mol. Bio. 48:443(1970*);* Pearson and Lipman, Methods in Mol. Biol. 24: 307-31(1988); Higgins and Sharp, Gene 73:237-44(1988); Higgins and Sharp, CABIOS 5:151-3(1989); Corpet et al., Nuc. Acids Res. 16:10881-90(1988); Huang et al., Comp. Appl. BioSci. 8:155-65(1992) and Pearson et al., Meth. Mol. Biol. 24:307-31 (1994). NCBI Basic Local Alignment Search Tool (BLAST)(Altschul et al., J. Mol. Biol. 215:403-10(1990)) is accessible from NBCI(National Center for Biological Information), and is available in conjunction with sequencing programs such as blastp, blastn, blastx, tblastn and tblastx on the Internet. BLAST is accessible through the BLAST page of the ncbi website. Sequence homology comparison methods using this program can be found on the BLAST help page of the ncbi website.

According to another aspect thereof, the present disclosure provides a recombinant vector comprising a nucleotide sequence encoding the polypeptide that binds to testosterone or the complex described above.

According to another aspect thereof, the present disclosure provides a host cell comprising the above-described recombinant vector.

In one embodiment of the present disclosure, the vector is operatively linked to the nucleotide sequence encoding the polypeptide that binds to testosterone or the complex described above.

As used herein, the term "operatively linked" refers to a functional linkage between a nucleic acid expression control sequence (e.g., promoter, signal sequence, or an array of transcriptional regulator binding sites) and another nucleic acid sequence, whereby the regulatory sequence regulates the transcription and/or translation of the other nucleic acid sequence.

The vector system of the present disclosure can be constructed through various methods known in the art, and specific methods thereof are disclosed in Sambrook, et al., Molecular Cloning, A Laboratory Manual, Cold Spring Harbor Laboratory Press (2001), which is incorporated herein by reference.

Vectors of the present disclosure can typically be constructed as vectors for cloning or as vectors for expression. In addition, the vector of the present disclosure may be constructed using a prokaryotic cell or a eukaryotic cell as a host.

For example, when the vector of the present disclosure is an expression vector and a prokaryotic cell is used as a host, it is common to include a strong promoter capable of propagating transcription (e.g., pLλ promoter, trp promoter, lac promoter, T7 promoter, tac promoter, and the like.), a ribosome binding site for initiation of translation, and a transcription/translation termination sequence. When *E. coli* is used as a host cell, the promoter and operator sites of the *E. coli* tryptophan biosynthesis pathway (Yanofsky, C., J. Bacteriol., 158:1018-1024(1984)) and the left-handed promoter of phage λ (pLλ promoter, Herskowitz, I. and Hagen, D., Ann. Rev. Genet., 14:399-445 (1980)) can be used as regulatory sites.

Meanwhile, vectors that can be used in the present disclosure can be produced by engineering plasmid (e.g., pJK hTx, pCDFduet, pCW57.1, pSK349, pSC101, ColE1, pBR322, pUC8/9, pHC79, pGEX series, pET series and pUC19, and the like.), phage (e.g., λgt·λ4B, λ-Charon, λΔz1, and M13, and the like.) or virus (e.g., SV40, and the like.), that are often used in the art.

The vector of the present disclosure can be fused with other sequences to facilitate purification of the polypeptides expressed therefrom. The sequence to be fused includes, for example, glutathione S-transferase (Pharmacia, USA), maltose binding protein (NEB, USA), FLAG (IBI, USA) and 6x His (hexahistidine; Quiagen, USA)., and the like. Because of the additional sequences for purification, the protein expressed in the host is rapidly and easily purified via affinity chromatography.

The vector of the present disclosure may include an antibiotic resistance gene commonly used in the art as a selection marker, and examples thereof include resistance genes to ampicillin, gentamicin, carbenicillin, chloramphenicol, streptomycin, kanamycin, geneticin, neomycin and tetracycline.

Meanwhile, when the vector of the present disclosure is an expression vector and a eukaryotic cell is used as a host, promoters derived from the genome of mammalian cells (e.g., metallotionine promoter) or promoters derived from mammalian viruses (e.g., adenovirus late promoter, vaccinia virus 7.5K promoter, SV40 promoter, cytomegalovirus promoter and HSV tk promoter can be used, and generally have a polyadenylation sequence as a transcription termination sequence.

Optionally, the vector may further carry genes encoding reporter molecules (e.g., luciferase and -glucuronidase).

As a host cell capable of stably and continuously cloning and expressing the vector of the present disclosure, any host cell known in the art can be used, and examples thereof include *E. coli* strains such as *E. coli* Origami2, *E. coli* JM109, *E*. *coli* BL21 (DE3), *E. coli* RR1, *E. coli* LE392, *E. coli* B, *E. coli* X 1776, *E. coli* W3110, *E. coli* Lemo(DE3), *Bacillus* genus strains such as *Bacillus subtilis, Bacillus thuringiensis,* and Enterobacteria and strains such as *Salmonella typhimurium, Serratia marcesens,* and various *Pseudomonas* species.

Further, when the vector of the present disclosure is transformed into a eukaryotic cell, yeast (*Saccharomyce cerevisiae*), insect cells, animal cells (e.g., CHO cell lines (Chinese hamster ovary), W138, BHK, COS-7, 293, HepG2, 3T3, RIN and MDCK cell lines), and the like can be used as the host cell.

In one embodiment of the present disclosure, the host cell transformed with the vector of the present disclosure is *E. coli.* In a more specific embodiment of the present disclosure, the host cells transformed with the vector of the present disclosure are ER2537 *E. coli* and BL21 (DE3), but are not limited thereto.

The method of delivering the vector of the present invention into a host cell can, when the host cell is a prokaryotic cell, be carried out by the CaCl₂ method (Cohen, S.N. et al., Proc. Natl. Acac. Sci. USA, 9:2110-2114 (1973)), Hanahan Method (Cohen, S.N. et al., Proc. Natl. Acac. Sci. USA, 9:2110-2114 (1973); and Hanahan, D., J. Mol. Biol., 166:557-580 (1983)) and electroporation methods (Dower, W. J. et al., Nucleic. Acids Res., 16:6127-6145 (1988)), and the like. Further, when the host cell is a eukaryotic cell, the vector may be injected into the host cell by a microinjection method (Capecchi, M.R., Cell, 22:479 (1980)), a calcium phosphate precipitation method (Graham, F.L. et al., Virology, 52:456 (1973)), an electroporation method (Neumann, E. et al., EMBO J., 1:841 (1982)), a liposome-mediated transfection method (Wong, T.K. et al., Gene, 10:87 (1980)), a DEAE-dextran treatment method (Gopal, Mol. Cell Biol., 5:1188-1190 (1985)), and a gene bombardment method (Yang et al., Proc. Natl. Acad. Sci., 87:9568-9572 (1990)), or the like.

In the present disclosure, a recombinant vector injected into the host cell can express the polypeptide or the complex recombined in the host cell, and in this case, a large amount of the polypeptide or the complex is obtained. For example, when the expression vector includes a lac promoter, host cells can be treated with IPTG to induce gene expression.

Cultivation of the transformed host cell can be performed by known host cell cultivation methods or modified methods thereof. For example, when the host cell is *E. coli,* the medium for culturing the transformed host cell includes a carbon source, a nitrogen source, an inorganic salt, and the like. that can be efficiently used by *E. coli,* and a natural medium or a synthetic medium may be used. Carbon sources that can be used include carbohydrates such as glucose, fructose, sucrose; starch, starch hydrolyzate; organic acids such as acetic acid and propionic acid; alcohols such as ethanol, propanol, and glycerol, and the like. The nitrogen source includes ammonia; ammonium salts of inorganic or organic acids such as ammonium chloride, ammonium sulfate, ammonium acetate and ammonium phosphate; peptone, meat extract, yeast extract, corn steep liquor, casein hydrolyzate, soybean extract, soybean hydrolyzate; various fermented cells and their decomposition products, and the like. The inorganic salt includes potassium dihydrogen phosphate, dipotassium hydrogen phosphate, magnesium phosphate, magnesium sulfate, sodium chloride, manganese sulfate, copper sulfate, calcium carbonate, and the like.

Cultivation is typically performed under aerobic conditions, such as by shaking culture or spinning on a rotator. The cultivation temperature is preferably in the range of 10°C to 40°C, and the cultivation time is generally 5 hours to 7 days. The pH of the medium is preferably maintained in the range of 3.0 to 9.0 during cultivation. The pH of the medium can be adjusted with inorganic or organic acids, alkaline solutions, urea, calcium carbonate, ammonia, and the like. During cultivation, antibiotics such as ampicillin, streptomycin, chloramphenicol, kanamycin and tetracycline may be added as needed for maintenance and expression of the recombinant vector. When culturing a host cell transformed with a recombinant expression vector having an inducible promoter, a suitable inducer can be added to the medium as needed. For example, when the expression vector contains the lac promoter, IPTG (isopropyl-β-D-thiogalactopyranoside) can be added, and when the expression vector contains the trp promoter, indoleacrylic acid can be added to the medium.

According to one aspect of thereof, the present disclosure provides a pharmaceutical composition for stimulating hair growth or treating hair loss, comprising: (a) the polypeptide that specifically binds to testosterone described above; and (b) a pharmaceutically acceptable excipient, carrier, or diluent.

According to another aspect thereof, the present disclosure provides a pharmaceutical composition for stimulating hair growth or treating hair loss, comprising: (a) the complex comprising a polypeptide that specifically binds to testosterone and at least one additional polypeptide described above; and (b) a pharmaceutically acceptable excipient, carrier, or diluent.

According to one aspect thereof, the present disclosure provides a cosmetic (aesthetic) composition for stimulating hair growth, which comprises the polypeptide that specifically binds to testosterone or the complex described above.

According to another aspect of the present disclosure, the present disclosure provides a cosmetic (aesthetic) composition for stimulating hair growth, which comprises the complex comprising a polypeptide that specifically binds to testosterone and at least one additional polypeptide described above.

i) The polypeptide that specifically binds to testosterone; or ii) the complex comprising the polypeptide that specifically binds to testosterone and at least one additional polypeptide, which is an active ingredient of the pharmaceutical/cosmetic composition for stimulating hair growth or treating hair loss of the present disclosure, can be synthesized by known means, such as liquid and solid phase synthesis (e.g., t-Boc solid phase peptide synthesis and BOP-SPPS).

It will be further appreciated by those skilled in the art that the present disclosure also includes a pharmaceutically and/or cosmetically acceptable acid or base addition salts of i) the polypeptide that specifically binds to testosterone; or ii) the complex comprising a polypeptide that specifically binds to testosterone and at least one additional polypeptide. The acids used to prepare the pharmaceutically and/or cosmetically acceptable acid addition salts of the aforementioned base compounds useful in the present disclosure are non-toxic acid addition salts, i.e., salts containing pharmaceutically and/or cosmetically acceptable anions such as, inter alia, hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate (i.e., 1,1'-methylene-bis-(2-hydroxy-3 naphthoate)) salts. Pharmaceutically and/or cosmetically acceptable base addition salts may also be used to produce pharmaceutically and/or cosmetically acceptable salt forms of the polypeptide or the complex.

The polypeptide or the complex of the present disclosure may be lyophilized for storage and reconstituted with an appropriate carrier prior to use.

The polypeptide or the complex is provided in the form of a composition comprising a pharmaceutically acceptable and/or cosmetically acceptable excipient, carrier or diluent selected with respect to the intended route of administration and standard pharmaceutical or cosmetic practice (e.g., Remington: The Science and Practice of Pharmacy, 19th edition, 1995, Ed. Alfonso Gennaro, Mack Publishing Company, Pennsylvania, USA, incorporated herein by reference).

"Pharmaceutically acceptable" means that the formulation is sterile and pyrogen free. Suitable pharmaceutical carriers are well known in the pharmaceutical arts. The carrier(s) should be "acceptable" in the sense that it does not adversely affect the efficacy of the active ingredient of the present disclosure and is not harmful to its recipient. Typically, the carrier will be sterile and pyrogen-free water or saline; however, other acceptable carriers may be used. Thus, "a pharmaceutically acceptable carrier" and "a pharmaceutically acceptable excipient" comprise the compound(s) used to form part of the formulation that is intended to simply act as a carrier, i.e., not intended to have its own biological activity. Pharmaceutically acceptable carriers or excipients are generally safe and non-toxic. Pharmaceutically acceptable carriers and/or excipients as used herein include all of one or more carriers and/or excipients.

Likewise, the term "cosmetically acceptable" is used to refer to an agent suitable for use as a cosmetic. Suitable cosmetic carriers are well known in the art, such as those commonly used in shampoos, lotions, creams, sprays and other such products.

The excipient may be one or more carbohydrates, polymers, lipids, and minerals. Examples of carbohydrates include lactose, sucrose, mannitol, and cyclodextrin, which are added to the composition to facilitate lyophilization, for example. Examples of polymers are starch, cellulose ether, cellulose carboxymethylcellulose, hydroxypropyl methyl cellulose, hydroxyethyl cellulose, ethyl hydroxyethyl cellulose, alginate, carrageenan, hyaluronic acid and its derivatives, polyacrylic acid, polysulfonates, polyethylene glycol/polyethylene oxide, polyethylene oxide/polypropylene oxide copolymers, polyvinyl alcohol/polyvinyl acetate of different degrees of hydrolysis, and polyvinylpyrrolidone of all different molecular weights, which are added to the composition, for example, for viscosity control, for adhesion, or for protecting lipids from chemical and proteolytic degradation. Examples of lipids are fatty acids, phospholipids, phosphoric acids, mono-, di-, and triglycerides, ceramides, sphingolipids and glycolipids of all different acyl chain lengths and saturations, egg lecithin, soybean lecithin, hydrogenated eggs and soybean lecithin, which are added to the composition for reasons similar to those for the polymer. Examples of minerals are talc, magnesium oxide, zinc oxide and titanium oxide, which are added to the composition to achieve advantages such as reduction of liquid accumulation or advantageous pigment properties.

The term "diluent" is intended to mean an aqueous or non-aqueous solution having the purpose of diluting a peptide in a pharmaceutical preparation. The diluent may be one or more saline, water, polyethylene glycol, propylene glycol, ethanol or oils (such as safflower oil, corn oil, peanut oil, cottonseed oil or sesame oil).

The diluent may also function as a buffer. The term "buffer" is intended to mean an aqueous solution containing an acid-base mixture with the purpose of stabilizing the pH. Examples of buffers are Trizma, Bicine, Tricine, MOPS, MOPSO, MOBS, Tris, Hepes, HEPBS, MES, phosphate, carbonate, acetate, citrate, glycolate, lactate, borate, ACES, ADA, tartarate, AMP, AMPD, AMPSO, BES, CABS, cacodylate, CHES, DIPSO, EPPS, ethanolamine, glycine, HEPPSO, imidazole, imidazolactic acid, PIPES, SSC, SSPE, POPSO, TAPS, TABS, TAPSO and TES.

Optionally, the composition may comprise an adjuvant. The term "adjuvant" is intended to mean a compound added to an agent to increase the biological effects of the peptide. Such adjuvants may be one or more zinc, copper or silver salts having different anions, such as, but not limited to, fluorides, chlorides, bromides, iodides, thiocyanates, sulfates, hydroxides, phosphates, carbonates, lactates, glycolates, citrates, borates, tartrates, and acetates of other acyl compositions.

The pharmaceutical compositions of the present disclosure may also be in the form of biodegradable microspheres. Aliphatic polyesters, such as poly(lactic acid) (PLA), poly(glycolic acid) (PGA), copolymers of PLA and PGA (PLGA) or poly(carprolactone) (PCL), and polyanhydrides, have been widely used as biodegradable polymers in the production of microspheres. Preparation of such microspheres can be found in in U.S. Pat. No. 5,851,451 and in EP0213303.

The pharmaceutical composition of the present disclosure may also be in the form of a polymer gel, or microneedle made of a polymer, wherein the polymer is starch, cellulose ether, cellulose carboxymethylcellulose, hydroxypropylmethyl cellulose, hydroxyethyl cellulose, ethylhydroxyethyl cellulose, alginate, carrageenan, hyaluronic acid and its derivatives, polyacrylic acid, polysulfonate, and the like.

The polypeptide that binds to testosterone or the complex of the present disclosure may be formulated in various concentrations, depending on the potency or toxicity of the active ingredient used. Preferably, the composition comprises the polypeptide that binds to testosterone or the complex of the present disclosure at a concentration of between 1 nM and 1 M, for example between 0.1 µM (micromole) and 1 mM, between 1 µM and 100 µM, between 5 µM and 50 µM, between 10 µM and 50 µM, between 20 µM and 40 µM and optionally about 30 µM. For *ex vivo* and *in vitro* applications, the composition may comprise a lower concentration of testosterone-binding polypeptide or complex, e.g., from 0.0025 µM to 1 µM.

It has been recognized by those skilled in the art that the compositions of the present disclosure can be administered by a variety of routes, including topical, subcutaneous, parenteral or oral administration.

Preferably, the compositions of the present disclosure are suitable for topical administration or intracutaneous administration. Thus, the composition of the present disclosure may be administered locally to the skin (e.g., the scalp). For example, the composition may be provided in the form of an ointment containing active polypeptide suspended or dissolved in a mixture having one or more of the following: mineral oil, liquid petrolatum, white petrolatum, propylene glycol, polyoxyethylene polyoxypropylene compounds, emulsifying waxes and water. Alternatively, the polypeptide may be formulated into, for example, an appropriate lotion or cream, suspended or dissolved in a mixture of one or more of the following: mineral oil, sorbitan monostearate, polyethylene glycol, liquid paraffin, polysorbate 60, cetyl ester wax, cetearyl alcohol, 2-octyldodecanol, benzyl alcohol and water.

Optionally, the composition for topical administration may comprise a penetration enhancer (e.g., as described in Osborne & Henke, 1997, Pharmaceutical Technology, November: 58-82 and Pathan & Setty, 2009, Tropical Journal of Pharmaceutical Research 8(2): 173-179, the disclosure of which is incorporated herein by reference).

Alternatively, the compositions of the present disclosure may be administered parenterally, e.g., intracutaneously. Such compositions are best used in the form of sterile aqueous solutions that may contain sufficient salts or glucose to make solutions isotonic with other substances, such as blood. If necessary, the aqueous solution above should, be buffered appropriately (preferably to a pH of 3-9). The preparation of suitable parenteral formulations under sterile conditions is readily accomplished by standard pharmaceutical techniques well known to those skilled in the art.

Compositions suitable for parenteral administration include aqueous and non-aqueous sterile injection solutions that may contain antioxidants, buffers, bacteriostats and solutes that make the formulation isotonic with the blood of the intended recipient; and aqueous and non-aqueous sterile suspensions that may include suspending agents and thickening agents. The formulations may be presented in unit-dose or multi-dose containers, such as sealed ampoules and vials, and may be stored in a lyophilized state requiring only the addition of a sterile liquid carrier, such as water for injection, immediately prior to use. Instant injection solutions and suspensions can be prepared from sterile powders, granules and tablets of the kind described above.

To deliver the polypeptide that binds to testosterone or the complex described above, it may be advantageous to use a sustained release system, such as a microspheres agent.

Alternatively, the composition may be administered by a device implanted by surgery to release the active polypeptide directly to the desired site (i.e., epidermis).

The composition of the present disclosure may also be delivered in a transdermal manner.

For example, an electroporation therapy (EPT) and/or iontophoresis system may be employed for administration of a protein or polypeptide. In such methods, the device is used to deliver a pulsed electric field to the cells, causing an increased permeability of the cell membrane to the drug and a significant increase of intracellular drug delivery.

An alternative transdermal method, electroincorporation, utilizes that small particles up to 30 microns in diameter on the surface of the skin experience the same or similar electrical pulses as used for electroporation. The particles enter the deeper layer of skin through the stratum corneum. The particles may be filled or coated with a drug or gene, or simply act as "bullets" that create pores in the skin through which the drug can enter.

Additional transdermal methods have also been developed by PowderJect Pharmaceuticals (now owned by Novartis AG).

Suitable methods for administration of the polypeptides and compositions of the present disclosure are well known in the art and see, for example, Therapeutic Protein and Peptide Formulation and Delivery, Zahra Shahrokh et al. (Eds), 1997, American Chemical Society, ISBN13: 978084123281.

The polypeptide composition of the present disclosure is administered to a subject in an effective amount. As used herein, a "therapeutically effective amount", "effective amount", or "therapeutically effective" refers to an amount that provides a stimulating effect on hair growth. This is a predetermined amount of the active substance calculated to produce the desired therapeutic effect. As will be appreciated by those skilled in the art, the amount of the active ingredient may vary depending on its specific activity. An appropriate dose may contain a predetermined amount of active substance calculated to produce the desired therapeutic effect with the required diluent. In the methods and uses for the preparation of the compositions of the present disclosure, a therapeutically effective amount of an active ingredient is provided. The therapeutically effective amount may be determined by a person skilled in the medical or veterinary arts based on patient characteristics such as age, weight, sex, condition, complications, other diseases, and the like, as is well known in the art.

In one embodiment of the present disclosure, said hair loss is selected from the group consisting of:
(a) androgenic alopecia (also known as androgenic hair loss, Alopesia androgenetica, male baldness or female baldness); (b) traction alopecia; (c) anagen effluvium (growth phase hair loss); (d) telogen effluvium(resting phase hair loss); (e) alopecia areata; (f) alopecia totalis (frontal hair loss); (g) alopecia universalis (systemic hair loss); (h) folliculitis alopecia (folliculitis hair loss); (i) alopecia mucinosa (mucinous hair loss); (j) neoplastic alopecia (neoplastic hair loss); (k) cicatricial alopecia (scarring alopecia); and (I) scarring hair loss.

For example, the hair loss may be androgenenic alopecia (androgenic hair loss).

Alternatively, the hair loss may be anagen effluvium (growth phase hair loss), e.g., induced by radiotherapy and/or chemotherapy (see above).

In one embodiment of the present disclosure, the subject is a mammal such as a human, a chimpanzee, an orangutan, a non-human primate, a dog, a cat, a horse, a hamster, a mouse, a rat, a gerbil, a pig, a sheep, a cow, and the like, and most particularly, a human, but is not limited thereto.

It is recognized by skilled people in the art in the art that the cosmetic compositions of the present disclosure are not limited to medical use and can be used as cosmetic agents (in the sense that they do not provide any physical health improvement, but merely provide aesthetic benefits to mammals).

Accordingly, the cosmetic composition may be for stimulating existing pores and/or inducing the growth of new pores (or stem cells capable of producing them).

In one specific embodiment, the cosmetic composition is used for the treatment or prophylaxis of bald head, which may be associated with a receding hairline and/or thinning hair.

Such compositions are not limited to use in the scalp and may be applied anywhere in the body (including the face to promote the growth of the beard, eyelashes or eyebrows).

It will be appreciated by those skilled in the art that the compositions of the present disclosure may be used alone or in combination with other therapeutic or cosmetic agents. For example, the compositions of the present disclosure can be used in combination therapy with existing therapies to prevent existing hair loss and/or stimulating new hair growth, e.g., in combination therapy with potassium channel openers such as Minoxidil (Regaine RTM., Pharmacia Corp.) and diazoxide; 5-alpha-reductase inhibitors such as Finasteride (Propecia RTM., Merck & Co.); and the immunosuppressant cyclosporin A.

It is also recognized by those skilled in the art that the compositions of the present disclosure can be used *in vivo, ex vivo* or *in vitro.*

Thus, in addition to being applied or administered directly to a mammal, the compositions can be used to stimulate hair growth *ex vivo,* e.g., in a skin explant prior to transplantation into the skin of a mammal.

Alternatively, the composition can be used to grow the pores *in vitro,* e.g., in cell culture, which can then be transplanted into the patient.

Accordingly, a further aspect of the present disclosure provides the use of polypeptides to stimulate hair growth *in vitro* or *ex vivo.*

In one embodiment of the present disclosure, the polypeptide is used to stimulate the growth of pores (or stem cells capable of producing it, dermal papilla cell).

According to another aspect thereof, the present disclosure provides a method of treating hair loss or stimulating hair growth, which comprises administering the above-described pharmaceutical composition to a subject in need thereof.

In one embodiment of the present disclosure, the pharmaceutical composition comprises the above-described polypeptide that specifically binds to testosterone as an active ingredient, or the above-described complex comprising one of the above-described polypeptides that specifically binds to testosterone and at least one angiogenic polypeptide as an active ingredient.

The subject of the present disclosure is a mammal such as a human, a chimpanzee, an orangutan, a non-human primate, a dog, a cat, a horse, a hamster, a mouse, a rat, a gerbil, a pig, a sheep, a cow, and the like, and most particularly a human, but is not limited thereto.

Since the method of treating hair loss or stimulating hair growth according to the present disclosure is a method including administering the compositions according to an aspect of the present disclosure, duplicate contents are omitted to avoid excessive complication of the present specification.

### Advantageous effects of the invention

The present disclosure relates to an affibody that binds to testosterone and uses thereof, and more specifically, to: i) an affibody that binds to testosterone or ii) a complex of the affibody and osteopontin fragment; and iii) a pharmaceutical composition for stimulating hair growth or treating hair loss which includes the same as an active ingredient. The affibody or the complex of the affibody and osteopontin fragment of the present disclosure has a very excellent effect of preventing hair growth or hair loss, and thus can be usefully used as a hair growth agent or a hair loss treatment agent.

### Brief description of the drawings

FIG. 1 is a diagram showing the results of alanine scanning of the affibody clone T26 that specifically binds to testosterone of the present disclosure.
FIG. 2 is a diagram showing the binding force with testosterone (T17-G-BSA) of the T26 variant, in which the threonine residue (T) at position 25 is replaced with another amino acid residue.
FIG. 3 is a diagram showing the results of SDS-PAGE analysis by purifying the T26 variant in which the threonine residue (T) at position 25 is replaced with another amino acid residue.
FIG. 4 is a diagram showing the selectivity of T26 variants for testosterone based on whether the selected T26 variants bind to four types of BSA (BSA, T3-CMO-BSA, G-BSA, T17-G-BSA).
FIG. 5 shows ELISA measurement values at various concentrations of Affibodies in a 4-parameter logistic curve which were measured to confirm affinity (EC₅₀) of the T26 and T26 variants of the present disclosure with testosterone (T17-G-BSA).
FIG. 6 shows the results of SDS-PAGE analysis of T26 and T26-INS001 of the present disclosure.
FIG. 7 shows the result of testing the binding of T26 and T26-INS001 to BSA, glycine-conjugated BSA (G-BSA) and testosterone-conjugated BSAs (T3-CMO-BSA, T17-G-BSA) to examine the testosterone binding selectivity of T26 and T26-INS001 of the present disclosure.
FIG. 8 is a diagram showing the affinity for testosterone of T26 and T26-INS001s having different sample storage periods which were tested to confirm the stability of T26-INS001 of the present disclosure. In FIG. 8, "the primary production" means a T26-INS001 sample previously produced, and "the secondary production" means a T26-INS001 sample produced after 30 days.
FIGS. 9a and 9b show the results of *in vivo* tests on the effects of T26 and T26-INS001 of the present disclosure on stimulating hair growth or treating hair loss.

### Mode for Carrying Out the Invention

Hereinafter, the present disclosure will be described in more detail with reference to examples. It would be obvious to those skilled in the art that these examples are intended to be more concretely illustrative and the scope of the present disclosure as set forth in the appended claims is not limited to or by the examples.

### EXAMPLE

Throughout the present specification, the "%" used to express the concentration of a specific material, unless otherwise particularly stated, refers to (wt/wt)% for solid/solid, (wt/vol)% for solid/liquid, and (vol/vol)% for liquid/liquid.

### Example 1: Preparation of bovine serum albumin (BSA) to which testosterone is conjugated

### Preparation of T17-G-BSA

The 17-beta hydroxyl group of testosterone (TCI, cat. #.T0027) was esterified with Boc-glycine (GENERAY BIOTECH, cat. #.0167) to introduce an amine group, and was conjugted to BSA (Sigam-Aldrich, cat. #.A7030) by using a Sulfo-SMCC (sulfosuccinimidyl 4-(N-maleimidomethyl)cyclohexane-1-carboxylate) (Thermo Fisher, cat. #.22322) linker to prepare T17-G-BSA having a structure such as BSA-linker-glycine-17beta-hydroxy Testosterone.

As a control, an antigen in which glycine was conjugated to BSA by with a Sulfo-SMCC linker was prepared (G-BSA). Each of the reaction mixtures reacted with T17-G-BSA and G-BSA was subjected to buffer exchanged using diafiltration method (Sartorius, cat. #. VS2002), and the small molecule materials remaining without participating in the reaction were removed.

### Preparation of T3-CMO-BSA

T3-CMO (Testosterone-3-(O-carboxymethyl)oxime) (Sigma-Aldrich, cat. #. T8390) was conjugated to BSA by inducing an amide bond between the carboxyl group of T3-CMO and the primary amine of BSA (T3-CMO-BSA). The reaction mixture was buffer-exchanged using a diafiltration method (Sartorius, cat. #. VS2002), and small molecule materials remaining without participating in the reaction were removed.

### Example 2: Testosterone-specific affibody selection

The affibody that specifically binds to testosterone was selected by a bio-panning method using magnetic beads (Thermo Fisher, cat. #. 14301) immobilized with T17-G-BSA.

Magnetic beads immobilized with T17-G-BSA used for bio-panning were prepared as follows: 30 mg (2 × 10⁹) of the magnetic beads were prepared by washing three times with 2 mL of 0.1 M sodium phosphate buffer (pH 7.4). 600 µg of T17-G-BSA of Example 1 was mixed with 30 mg of washed magnetic beads, and 0.1 M sodium phosphate buffer was added so that the volume of the solution was 600 µL. In order to react the epoxy group of the magnetic bead with the amine group of T17-G-BSA, 300 µL of 3 M ammonium sulfate was added, and then the reaction was performed at 37°C for 24 hours.

Bio-panning screening for the selection of affibodies that specifically bind to testosterone was performed as follows: Affibody library was rescue in phage form using VCSM13 helper phage and used for panning. The number of library phage binding to the initial antigen was 10¹³ or more, and a panning of 5 round was performed. As a strategy for selectively selecting phages with high affinity, the amount of magnetic beads was reduced as the panning round order was increased (50 µg, 30 µg, 20 µg, 20 µg, and 10 µg, respectively). The number of washing was 3 times with PBST (phosphate buffered saline + 0.05% Tween 20) in the first round, and 5 times in the next round (2 to 4 rounds).

The number of phages bound to the target antigen was measured (titrated) using ER2537 *E. coli* as follows: Binder phages obtained from each round of bio-panning was eluted with a 0.1 M HCl solution containing 20 mM free testosterone. ER2537 *E. coli* cultured overnight in SB media (MOPS 10 g/L, Bacto YEAST extract 20 g/L, Trypton 30 g/L), was passaged by 1/200 dilution in new SB media, and further cultured for 3 hours at 37°C to reach log phase. 100 µL of fresh ER2537 *E*. *coli* and 10 µL of the diluted phage were mixed in a 1.5 mL unit tube, incubated for 30 minutes, and then spread on an LB plate containing ampicillin, and cultured at 37°C for 16 hours. The number of phages was measured by applying the number of generated colonies of ER2537 *E. coli* and a dilution factor.

The binder phages obtained from each round of bio-panning was infected with ER2537 *E. coli* and maintained in a colony form. Binding to each antigen was confirmed by ELISA as follows: ER2537 *E. coli* colony obtained by infection with Binder phage was inoculated to SB media and cultured until 0.5 at OD₆₀₀. The affibody expression induction was performed by adding 0.5 mM IPTG (Elpis Solution) and shaking at 30°C for 16 hours. The fraction of the intermembrane space of ER2537 *E. coli* was extracted using BBS buffer (200 mM Boric acid, 150 mM NaCl, 1 mM EDTA, pH 7.5). The affibody extraction fractions were treated with ELISA plate coated with 0.2 mg/well of T17-G-BSA at room temperature for 1 hour, and then washed three times with TBST (Tris buffered saline+0.05% Tween 20). The binding of the affibody to the antigen was examined by treating anti-HA mouse HRP (Roche, cat. #. 12013819001) that binds to the HA (hemagglutinin) tag of the C-terminus of the expressed affibody as a secondary antibody at room temperature for 1 hour, washing it three times with TBST, developing it with TMB (3', 5, 5'-Tetramethylbenzidine) solution, and measuring an OD₄₅₀ value using ELISA reader (Victor X3 PerkinElmer).

Competitive ELISA was performed to select affibody capable of specific binding to the free testosterone, which is the purpose of the present disclosure. The affibody extraction fractions were pre-treated with 0.1 mM free testosterone at room temperature for 1 hour, and then the above ELISA procedure was repeated. As a result, an affibody (T26) that specifically binds to both T17-G-BSA and free testosterone was identified. Phagemid plasmid thereof was obtained and nucleic acid sequence analysis thereof was performed. The results are shown in Table 1.

**Table 1. Affibodies selected by bio-Panning and sequences of affibodies derived by affinity maturation**

| Classi ficatio n | Nucleotide sequences | Amino acid sequences |
|---|---|---|
| T26(P arenta l) | GTGGACAACAAGTTCAACAAGGAGATGGTTCAGGCCATG CGTGAGATTTCTTACCTGCCCAACCTGAACCATACGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 23) | VDNKFNKEM VQAMREISYL PNLNHTQIRA FIWVLFDDPS QSANLLAEAK KLNDAQAPK (SEQ ID NO: 1) |
| T26-Q11A | GTGGACAACAAGTTCAACAAGGAGATGGTTGCTGCCATG CGTGAGATTTCTTACCTGCCCAACCTGAACCATACGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 24) | VDNKFNKEM VAAMREISYL PNLNHTQIRA FIWVLFDDPS QSANLLAEAK KLNDAQAPK (SEQ ID NO: 2) |
| T26-S17A | GTGGACAACAAGTTCAACAAGGAGATGGTTCAGGCCATG CGTGAGATTGCTTACCTGCCCAACCTGAACCATACGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 25) | VDNKFNKEM VQAMREIAYL PNLNHTQIRA FIWVLFDDPS QSANLLAEAK KLNDAQAPK (SEQ ID NO: 3) |
| T26-T25A | GTGGACAACAAGTTCAACAAGGAGATGGTTCAGGCCATG CGTGAGATTTCTTACCTGCCCAACCTGAACCATGCTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 26) | VDNKFNKEM VQAMREISYL PNLNHAQIRA FIWVLFDDPS QSANLLAEAK KLNDAQAPK (SEQ ID NO: 4) |
| T26-T25R | GTGGACAACAAGTTCAACAAGGAGATGGTTCAGGCCATG CGTGAGATTTCTTACCTGCCCAACCTGAACCATAGGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 27) | VDNKFNKEM VQAMREISYL PNLNHRQIRA FIWVLFDDPS QSANLLAEAK KLNDAQAPK (SEQ ID NO: 5) |
| T26-T25H | GTGGACAACAAGTTCAACAAGGAGATGGTTCAGGCCATG CGTGAGATTTCTTACCTGCCCAACCTGAACCATCATCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 28) | VDNKFNKEM VQAMREISYL PNLNHHQIRA FIWVLFDDPS QSANLLAEAK KLNDAQAPK (SEQ ID NO: 6) |
| T26-T25K | GTGGACAACAAGTTCAACAAGGAGATGGTTCAGGCCATG CGTGAGATTTCTTACCTGCCCAACCTGAACCATAAGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 29) | VDNKFNKEM VQAMREISYL PNLNHKQIRA FIWVLFDDPS QSANLLAEAK KLNDAQAPK (SEQ ID NO: 7) |
| T26-T25D | GTGGACAACAAGTTCAACAAGGAGATGGTTCAGGCCATG CGTGAGATTTCTTACCTGCCCAACCTGAACCATGATCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 30) | VDNKFNKEM [?] VQAMREISYL PNLNHDQIRA FIWVLFDDPS QSANLLAEAK KLNDAQAPK (SEQ ID NO: 8) |
| T26-T25E | GTGGACAACAAGTTCAACAAGGAGATGGTTCAGGCCATG CGTGAGATTTCTTACCTGCCCAACCTGAACCATGAGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 31) | VDNKFNKEM [?] VQAMREISYL PNLNHEQIRA FIWVLFDDPS QSANLLAEAK KLNDAQAPK (SEQ ID NO: 9) |
| T26-T25S | GTGGACAACAAGTTCAACAAGGAGATGGTTCAGGCCATG CGTGAGATTTCTTACCTGCCCAACCTGAACCATAGTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 32) | VDNKFNKEM VQAMREISYL PNLNHSQIRA FIWVLFDDPS QSANLLAEAK KLNDAQAPK (SEQ ID NO: 10) |
| T26-T25N | GTGGACAACAAGTTCAACAAGGAGATGGTTCAGGCCATG CGTGAGATTTCTTACCTGCCCAACCTGAACCATAATCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 33) | VDNKFNKEM [?] VQAMREISYL PNLNHNQIRA FIWVLFDDPS QSANLLAEAK KLNDAQAPK (SEQ ID NO: 11) |
| T26-T25Q | GTGGACAACAAGTTCAACAAGGAGATGGTTCAGGCCATG CGTGAGATTTCTTACCTGCCCAACCTGAACCATCAGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 34) | VDNKFNKEM [?] VQAMREISYL PNLNHQQIRA FIWVLFDDPS QSANLLAEAK KLNDAQAPK (SEQ ID NO: 12) |
| T26-T25C | GTGGACAACAAGTTCAACAAGGAGATGGTTCAGGCCATG CGTGAGATTTCTTACCTGCCCAACCTGAACCATTGTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 35) | VDNKFNKEM VQAMREISYL PNLNHCQIRA FIWVLFDDPS QSANLLAEAK KLNDAQAPK (SEQ ID NO: 13) |
| T26-T25G | GTGGACAACAAGTTCAACAAGGAGATGGTTCAGGCCATG CGTGAGATTTCTTACCTGCCCAACCTGAACCATGGTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 36) | VDNKFNKEM VQAMREISYL PNLNHGQIRA FIWVLFDDPS QSANLLAEAK KLNDAQAPK (SEQ ID NO: 14) |
| T26-T25P | GTGGACAACAAGTTCAACAAGGAGATGGTTCAGGCCATG CGTGAGATTTCTTACCTGCCCAACCTGAACCATCCTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 37) | VDNKFNKEM VQAMREISYL PNLNHPQIRA FIWVLFDDPS QSANLLAEAK KLNDAQAPK (SEQ ID NO: 15) |
| T26-T25V | GTGGACAACAAGTTCAACAAGGAGATGGTTCAGGCCATG CGTGAGATTTCTTACCTGCCCAACCTGAACCATGTGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 38) | VDNKFNKEM VQAMREISYL PNLNHVQIRA FIWVLFDDPS QSANLLAEAK KLNDAQAPK (SEQ ID NO: 16) |
| T26-T25I | GTGGACAACAAGTTCAACAAGGAGATGGTTCAGGCCATG CGTGAGATTTCTTACCTGCCCAACCTGAACCATATTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 39) | VDNKFNKEM VQAMREISYL PNLNHIQIRA FIWVLFDDPS QSANLLAEAK KLNDAQAPK (SEQ ID NO: 17) |
| T26-T25L | GTGGACAACAAGTTCAACAAGGAGATGGTTCAGGCCATG CGTGAGATTTCTTACCTGCCCAACCTGAACCATCTTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 40) | VDNKFNKEM VQAMREISYL PNLNHLQIRA FIWVLFDDPS QSANLLAEAK KLNDAQAPK (SEQ ID NO: 18) |
| T26-T25F | GTGGACAACAAGTTCAACAAGGAGATGGTTCAGGCCATG CGTGAGATTTCTTACCTGCCCAACCTGAACCATTTTCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 41) | VDNKFNKEM VQAMREISYL PNLNHFQIRA FIWVLFDDPS QSANLLAEAK KLNDAQAPK (SEQ ID NO: 19) |
| T26-T25Y | GTGGACAACAAGTTCAACAAGGAGATGGTTCAGGCCATG CGTGAGATTTCTTACCTGCCCAACCTGAACCATTATCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 42) | VDNKFNKEM[?] VQAMREISYL PNLNHYQIRA FIWVLFDDPS QSANLLAEAK KLNDAQAPK (SEQ ID NO: 20) |
| T26-T25M | GTGGACAACAAGTTCAACAAGGAGATGGTTCAGGCCATG CGTGAGATTTCTTACCTGCCCAACCTGAACCATATGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 43) | VDNKFNKEM VQAMREISYL PNLNHMQIR AFIWVLFDDP SQSANLLAEA KKLNDAQAP K (SEQ ID NO: 21) |
| T26-T25W | GTGGACAACAAGTTCAACAAGGAGATGGTTCAGGCCATG CGTGAGATTTCTTACCTGCCCAACCTGAACCATTGGCAGA TCCGTGCCTTCATTTGGGTTCTGTTCGACGACCCTTCCCA GTCCGCCAACCTGCTGGCCGAGGCCAAGAAGCTGAACGA CGCCCAGGCTCCCAAG (SEQ ID NO: 44) | VDNKFNKEM VQAMREISYL PNLNHWQIR AFIWVLFDDP SQSANLLAEA KKLNDAQAP K (SEQ ID NO: 22) |

### Example 3: T26 affinity maturation

Alanine screening of the variable region was performed to identify the region involved in the binding of the selected T26 affibody and testosterone. Based on the selected sequence of T-26, primers in which the sequence of each variable region can be replaced with alanine were designed. GCT was used as the alanine codon. The alanine sequence replacement method was performed by the over-lapping PCR method as follows. To generate N-terminal DNA fragments based on the variable region amino acid to be mutated, 50 ng of a template DNA, 4 µL of #22 forward primer and variable region reverse primer at a concentration of 10 pmol/µL, respectively, 10 µL 10X pfu polymerase mixture (ELPIS biotech, EBT-1011) were added to a PCR tube. Distilled water was added so that the total reaction volume became 50 µL, and then PCR was performed. In addition, based on the variable region amino acid to be mutated, the C-terminal side DNA fragment was generated using the variable region forward and #24 reverse primers under the same conditions as above. After loading the two DNA fragments generated by the PCR reaction on an agarose gel, DNA was separated from the agarose gel using a DNA gel elution kit (iNtRON BIOTECHNOLOGY, cat. #. 17288). After putting 50 ng of each of the two separated DNA fragments, 4 µL of #22 forward primer and #24 reverse at a concentration of 10 pmol/µL, respectively, 10 µL 10X pfu polymerase mixture (ELPIS biotech, EBT-1011) were added to a PCR tube. Distilled water was added so that the total reaction volume became 50 µL, and then overlapping PCR was performed to link the two DNA fragments, thereby preparing a variant sequence in which amino acid at each variable region was replaced with alanine. (See primer sequences in Table 2)

**Table 2. Primer sequences used in the screening of alanine T26 affibodies**

| Primers | Nucleotide sequence (5' to 3') | SEQ ID NO: |
|---|---|---|
| #22 forward | gtgtggaattgtgagcggataac | 45 |
| #24 reverse | atagcccccttattagcgtttgccatc | 46 |
| M9A-forward | CAAGTTCAACAAGGAGGCTGTTCAGGCCATG CGTG | 47 |
| V10A-forward | GTTCAACAAGGAGATGGCTCAGGCCATGCGT GAGA | 48 |
| Q11A-forward | CAACAAGGAGATGGTTGCTGCCATGCGTGAG ATTTC | 49 |
| M13A-forward | GGAGATGGTTCAGGCCGCTCGTGAGATTTCTT ACC | 50 |
| R14A-forward | GATGGTTCAGGCCATGGCTGAGATTTCTTACC TGC | 51 |
| S17A-forward | CAGGCCATGCGTGAGATTGCTTACCTGCCCA ACCTGAAC | 52 |
| Y18A-forward | CATGCGTGAGATTTCTGCTCTGCCCAACCTGA ACC | 53 |
| H24A-forward | CTTACCTGCCCAACCTGAACGCTACTCAGATC CGTGCCTTC | 54 |
| T25A-forward | _ CTGCCCAACCTGAACCATGCTCAGATCCGTG CCTTCAT | 55 |
| I27A-forward | CAACCTGAACCATACTCAGGCTCGTGCCTTCA TTTGGGTTC | 56 |
| R28A-forward | CTGAACCATACTCAGATCGCTGCCTTCATTTG GGTTCTG | 57 |
| W32A-forward | CAGATCCGTGCCTTCATTGCTGTTCTGTTCGA CGACCCTT | 58 |
| V33A-forward | GATCCGTGCCTTCATTTGGGCTCTGTTCGACG ACCCTTCC | 59 |
| F35A-forward | CCTTCATTTGGGTTCTGGCTGACGACCCTTCC CAGTC | 60 |

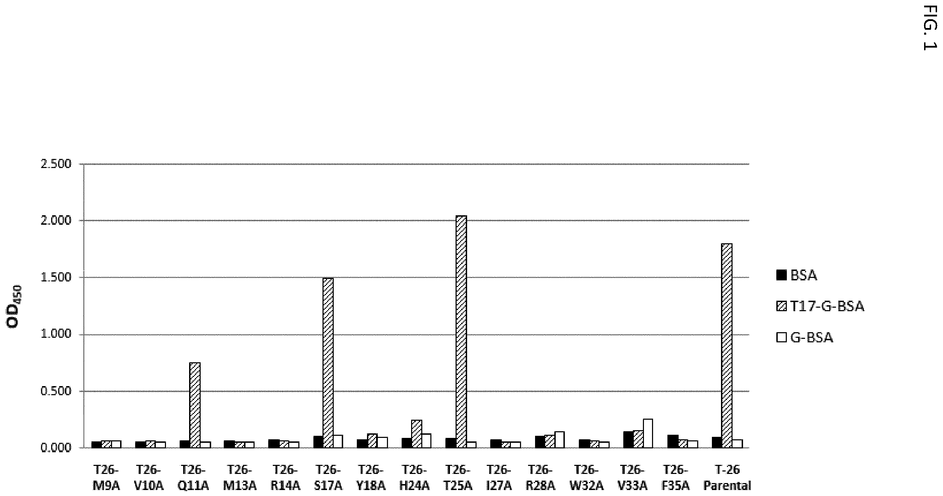

The intermembrane fractions of each clone were subjected to ELISA to confirm the binding specificity to the BSA, T17-G-BSA and G-BSA antigens. As a result, it was confirmed that binding and specificity with T17-G-BSA were maintained in three clones (T26-Q11A, T26-S17A and T26-T25A) in which the glutamine residue (Q) at position 11, the serine residue (S) at position 17, or the threonine residue (T) at position 25, respectively, in the T26 sequence was replaced with alanine (FIG. 1). The nucleotide sequence and amino acid sequence of clones for which T17-G-BSA binding ability was confirmed are shown in Table 1. In particular, from the fact that the binding ability of T25A to T17-G-BSA was increased to the parental clone (T26), the inventors expected that the replacement of the amino acid residue at position 25 could increase the affinity of T26 to testosterone. Affibodies in which the threonine residue at position 25 of T26 was replaced with another amino acid were prepared as follows. Based on the selected T26 sequence, the threonine residue at position 25 was replaced with another amino acid by over-lapping PCR using NNK primer. (See primer sequences in Table 3)

**Table 3. Primer sequences used for the T26-T25X mutation**

| Primers | Nucleotide sequence (5' to 3') | SEQ ID NO: |
|---|---|---|
| #22 forward | gtgtggaattgtgagcggataac | 45 |
| #24 reverse | atagcccccttattagcgtttgccatc | 46 |
| T25NNK-forward | CTGCCCAACCTGAACCATNNKCAGAT CCGTGCCTTCAT | 61 |
| T25-reverse | ATGGTTCAGGTTGGGCAG | 62 |

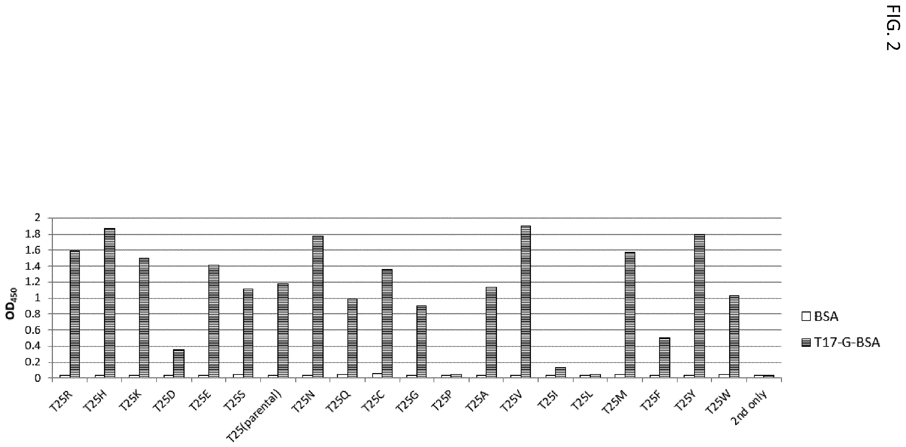

The nucleotide sequences and amino acid sequences of the affibodies in which the threonine residue at position 25 was replaced with other amino acids are shown in Table 1. The intermembrane fractions of each clone were subjected to ELISA using BSA and T17-G-BSA as antigens (FIG. 2). On the basis of affinity, 10 species of affibodies in which the threonine residue at position 25 was replaced with arginine (T26-T25R), histidine (T26-T25H), lysine (T26-T25K), asparagine (T26-T25N), alanine (T26-T25A), valine (T26-T25V), methionine (T26-T25M), tyrosine (T26-T25Y), tryptophan (T26-T25W), glutamine (T26-T25Q) were further selected. The genes of selected affibodies were amplified by PCR (see primer sequences in Table 4), treated with Ndel (NEB cat. # R0111L) and Xhol (NEB cat. # R0146L) restriction enzymes, and then cloned into pET21a (Novagen, cat. #. 69740) expression vector.

**Table 4. Primer used for cloning T26-T25X mutant pET21a**

| Primers | Nucleotide sequence (5' to 3') | SEQ ID NO: |
|---|---|---|
| forward | AATTCATATGGTGGACAACAAGTTCAACAAGGAG | 63 |
| reverse | CCGCTCGAGCTTGGGAGCCTGGGCGTCGTTC | 64 |

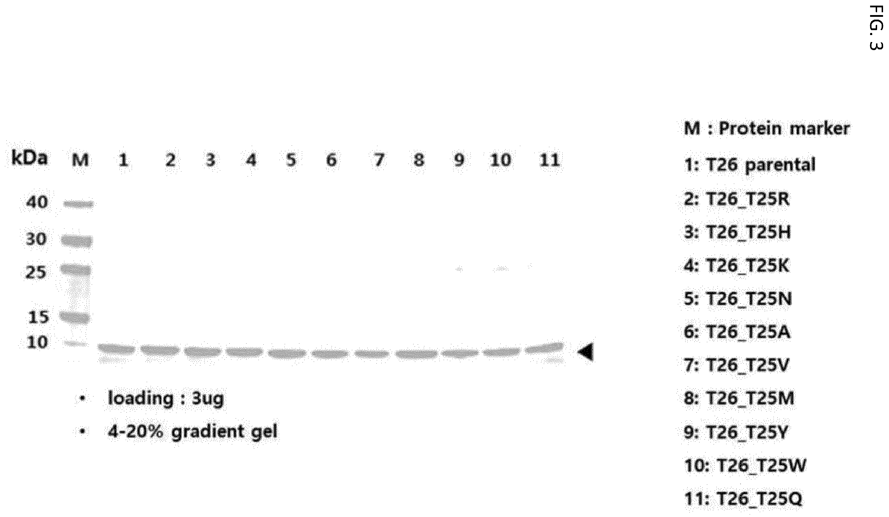

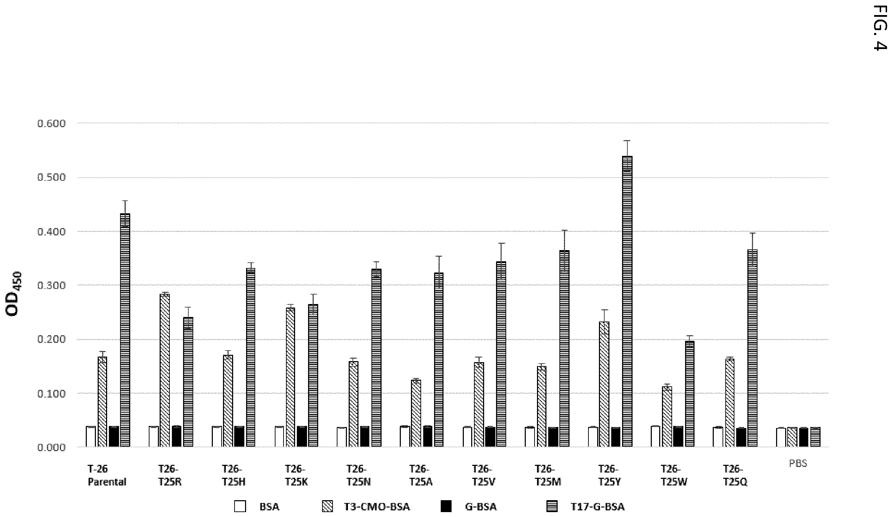

The transformed BL21 (DE3) colonies were inoculated into 5 mL 2X YT media (5 g/L of NaCl, 10 g/L of Bacto YEAST extract, 16 g/L of Trypton) containing 100 µg/mL of ampicillin, and pre-cultured at 37°C for 16 hours. 200 µL of the pre-culture solution was inoculated into 250 mL of 2X YT media and cultured until reaching 0.5 in 37°C OD₆₀₀. The affibody expression induction was performed by adding 1 mM IPTG and shaking culture at 30°C for 16 hours. The bacteria collected by centrifugation were suspended in lysis buffer (6 M guanidine, 47 mM Na₂HPO₄, 2.65 mM NaH₂PO₄, 10 mM Tris, 100 mM NaC!, pH8.0) and then subjected to ultrasonic disruption. The affibodies were purified from the lysate using Ni-NTA beads (Qiagen, cat. #. 30410) and replaced with PBS in a purification buffer using the diafiltration method. The purified Affibodies were quantified by measuring absorbance at 280 nm. The purified recombinant protein was analyzed by SDS-PAGE using 4-20% gradient gel (FIG. 3). 10 µg/mL (50 µL) of each purified affibody was tested for binding specificity using ELISA plate coated with BSA, T3-CMO-BSA, G-BSA, and T17-G-BSA at a concentration of 100 ng per well, respectively. The Affibodies were treated at room temperature for 1 hour and then washed three times with TBST. The antigen binding of affibodies was examined by anti-6x His mouse HRP (R&D systems, cat. #. MAB050H) as a secondary antibody at room temperature for 1 hour, washing with TBST three times, developing with TMB solution, and measuring the OD₄₅₀ value using ELISA reader (Victor X3 Perkin Elmer). As a result, 11 types of affibodies, including T26 were found to bind only to the antigen to which testosterone was conjugated, T3-CMO-BSA and T17-G-BSA, and not to BSA and G-BSA at all (FIG. 4).

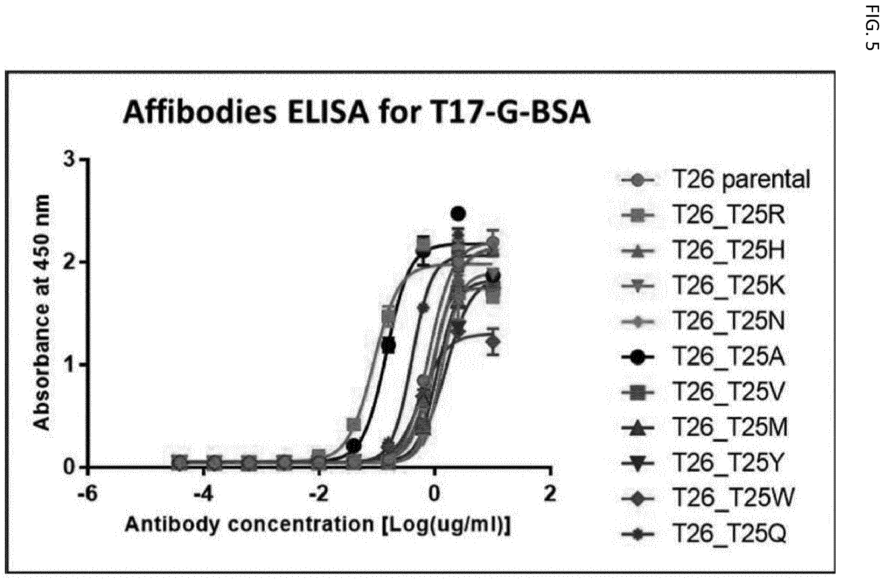

The binding force of each affibody to T17-G-BSA was confirmed by ELISA. On an ELISA plate coated with T17-G-BSA at a concentration of 100 ng per well, affibodies at a concentration of 40 µg/mL were serially diluted 5-fold, 10 times in a row, treated with a minimum concentration of 20.48 pg/mL at room temperature for 1 hour, and subjected to ELISA. The value of OD₄₅₀, which is the degree of color development of TMB solution, was put into the GraphPad Prism program, and the EC 50 value was calculated from 4-parameter logistic curve (FIG. 5 and Table 5). As a result, the EC₅₀ value for T17-G-BSA of T26 was calculated to be about 0.8 µg/mL (1 × 10⁻⁷ M), and T26-T25R (9.3 times affinity), T26-T25A (5.6 times affinity), and T26-T25Q (2.0 times affinity) were identified as affibodies with more improved affinity.

**Table 5**

| | | | | | | | | | | | |
|---|---|---|---|---|---|---|---|---|---|---|---|
| 2nd ELISA | T26 parental | T26-T25R | T26-T25H | T26-T25K | T26-T25N | T26-T25A | T26-T25V | T26-T25M | T26-T25Y | T26-T25W | T26-T25Q |
| EC₅₀(µg/mL) | 0.806 | 0.087 | 0.990 | 1.304 | 1.013 | 0.145 | 0.894 | 1.100 | 1.509 | 0.576 | 0.400 |
| Affinity | 1.0 times | 9.3 times | 0.8 times | 0.6 times | 0.8 times | 5.6 times | 0.9 times | 0.7 times | 0.5 times | 1.4 times | 2.0 times |

### Example 4. Production and characterization of affibody-angiogenic peptide fusion protein

The present inventors prepared a complex in which the T26 affibody and the fragment of osteopontin protein (SVYGLR) were linked by a peptide linker (GGGG) and named it T26-INS001. For ease of purification, the T26-INS001 was cloned into a pET28a expression vector with a 6X histidine tag upstream of the N-terminus of T26 and a thrombin cleavage sequence therebetween, as follows: a pET21a vector into which T26 was inserted was used as a PCR amplification template (see primer sequence in Table 6).

**Table 6. Primers used for cloning pET21a, protein expression vector for animal test samples (T26, T26-INS001)**

| Primers | Nucleotide sequence (5' to 3') | SEQ ID NO: |
|---|---|---|
| Forward | AATTCATATGGTGGACAACAAGTTCAACAAGGAG | 63 |
| reverse (T26) | CCGCTCGAGCTTGGGAGCCTGGGCGTCGTTC | 64 |
| reverse (T26-INS001) | CCGCTCGAGTTAGCGCAGGCCATACACCACGCTGCCGCCG CCGCCCTTGGGAGCCTGGGCGTCG | 65 |

The PCR amplification products were treated with Ndel (NEB cat. #. R0111L) and Xhol (NEB cat. #. R0146L) restriction enzymes and then inserted into a MCS (multiple cloning site) of pET28a (Novagen, cat. #. 69864). Nucleic acid sequences and amino acid sequences of each protein are shown in Table 7.

**Table 7. Sequence of a sample used in animal experiments**

| Classific ation | Nucleotide sequence | Amino acid sequence |
|---|---|---|
| T26 | ATGGGCAGCAGCCATCATCATCATCATCACAGCA GCGGCCTGGTGCCGCGCGGCAGCCATATGGTGG ACAACAAGTTCAACAAGGAGATGGTTCAGGCCAT GCGTGAGATTTCTTACCTGCCCAACCTGAACCATA CGCAGATCCGTGCCTTCATTTGGGTTCTGTTCGA CGACCCTTCCCAGTCCGCCAACCTGCTGGCCGAG GCCAAGAAGCTGAACGACGCCCAGGCTCCCAAGT AA (SEQ ID NO: 68) | MGSSHHHHHHSS GLVPRGSHMVDN KFNKEMVQAMREI SYLPNLNHTQIRAF IWVLFDDPSQSAN LLAEAKKLNDAQA PK (SEQ ID NO: 67) |
| T26-INS001 | ATGGGCAGCAGCCATCATCATCATCATCACAGCA GCGGCCTGGTGCCGCGCGGCAGCCATATGGTGG ACAACAAGTTCAACAAGGAGATGGTTCAGGCCAT GCGTGAGATTTCTTACCTGCCCAACCTGAACCATA CGCAGATCCGTGCCTTCATTTGGGTTCTGTTCGA CGACCCTTCCCAGTCCGCCAACCTGCTGGCCGAG GCCAAGAAGCTGAACGACGCCCAGGCTCCCAAG GGCGGCGGCGGCAGCGTGGTGTATGGCCTGCGC TAACTCGAGCACCACCACCACCACCACTGAGATC CGGCTGCTAA (SEQ ID NO: 70) | MGSSHHHHHHSS GLVPRGSHMVDN KFNKEMVQAMREI SYLPNLNHTQIRAF IWVLFDDPSQSAN LLAEAKKLNDAQA PKGGGGSVVYGL R (SEQ ID NO: 69) |

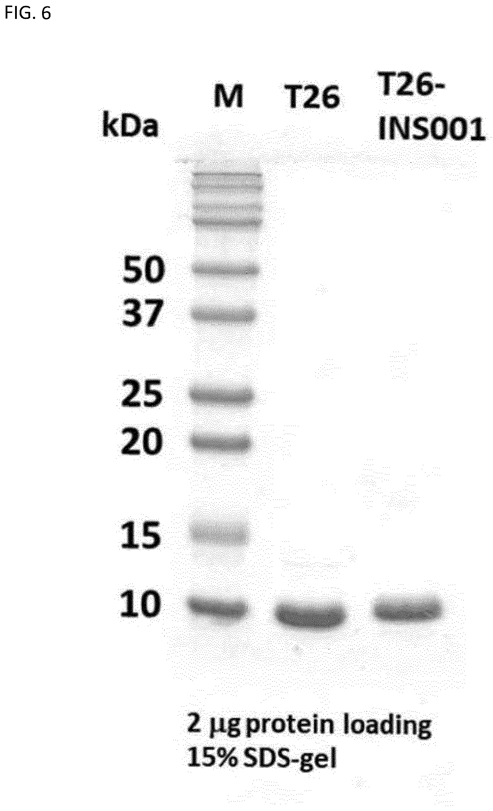

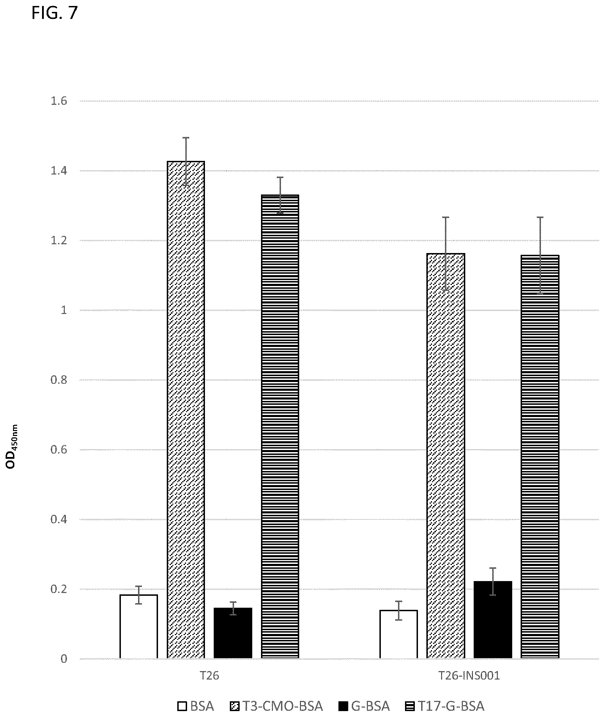

The transformed BL21 (DE3) colonies were inoculated into 5 mL 2X YT media (5 g/L of NaCl, 10 g/L of Bacto YEAST extract, 16 g/L of Trypton) containing 50 µg/mL of Kanamycin and pre-cultured at 37°C for 16 hours. 500 µL of the pre-culture solution was inoculated into 500 mL of 2X YT media, and cultured until until reaching 0.5 in 37°C OD₆₀₀. Expression of T26 (cloned into pET28 vector), T26-INS001 was induced by adding 1 mM IPTG and shaking culture at 25°C for 24 hours. The bacteria collected by centrifugation were suspended in lysis buffer (6 M guanidine, 47 mM Na₂HPO₄, 2.65 mM NaH₂PO₄, 10 mM Tris, 100 mM NaCl, pH8.0) and then subjected to ultrasonic disruption. T26 and T26-INS001 were purified from the lysate using Ni-NTA beads and replaced with saline (0.9% NaCl) in a purification buffer using the diafiltration method. The purified T26 and T-26-INS001 were quantified by measuring absorbance at 280 nm. The purified recombinant protein was analyzed by 15% SDS-PAGE (FIG. 6). The binding specificity of 0.1 µg/mL (50 µL) of purified T26, T26-INS001 was confirmed using an ELISA plate coated with BSA, T3-CMO-BSA, G-BSA, and T17-G-BSA at a concentration of 100 ng per well, respectively. T26 and T26-INS001 were treated at room temperature for 1 hour, and then washed three times with TBST. The antigen binding of T26 and T-26-INS001 was confirmed by treating anti-6x His mouse HRP as a secondary antibody at room temperature for 1 hour, washing it three times with TBST, performing a color reaction with TMB solution, and measuring an OD₄₅₀ value using ELISA reader (Victor X3 PerkinElmer). As a result, it was found that T26 and T26-INS001 bind only to the testosterone-conjugated antigens, T3-CMO-BSA and T17-G-BSA, but not to BSA and G-BSA (FIG. 7). It was confirmed that the N-terminal peptide (6X histidine tag, thrombin cleavage sequence) and the C-terminal peptide of T26 did not affect the binding specificity of T26.

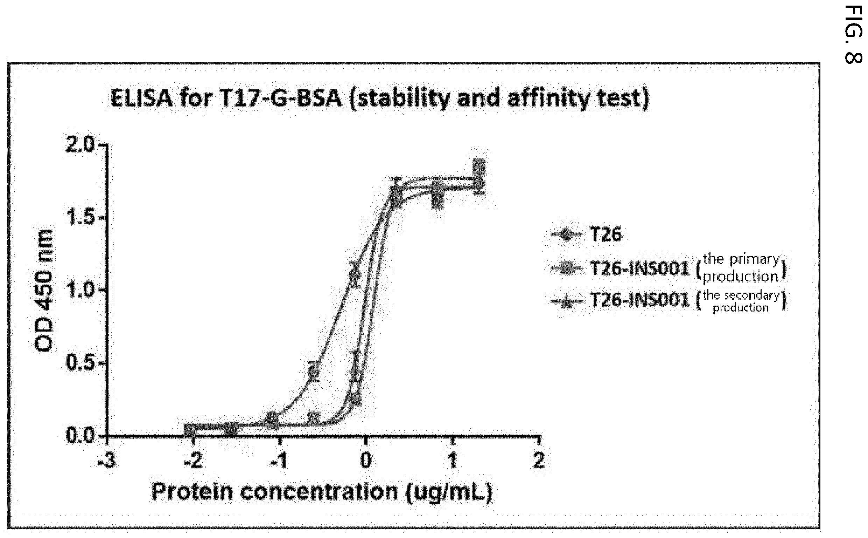

The binding affinity of T26, T26-INS001 to T17-G-BSA was confirmed by ELISA. On an ELISA plate coated with T17-G-BSA at a concentration of 100 ng per well, T26 and T26-INS001 were serially diluted at a concentration of 20 µg/mL (50 µL), 3-fold each for eight times in a row, treated with a minimum concentration of 9 ng/mL at room temperature for 1 hour, and subjected to ELISA. In order to test the stability of T26-INS001, ELISA was performed on each of the previously prepared samples (the primary production) and the newly prepared samples (the secondary production) prepared at 30-day intervals. The previously prepared T26-INS001 sample (the primary production) was stored at 4°C in a state of being dissolved in 0.9% NaCl physiological saline until the secondary production of T26-INS001 sample. The value of OD₄₅₀, which is the degree of color development of TMB solution, was put into the GraphPad Prism program, and the EC₅₀ value was calculated from 4-parameter logistic curve (FIG. 8). As a result, the EC₅₀ value for T17-G-BSA of T26 was calculated as 0.5 mg/l (6 × 10⁻⁹ M), and T26-INS001 was found to have an EC₅₀ value that was twice lower than that. Therefore, it was found that the peptide fused to the C-terminus had little effect on the affinity for T17-G-BSA of T26. In addition, it was confirmed that the stability of T26-INS001 could be maintained for at least 30 days because the difference in affinity between T26-INS001 having different sample preparation time was insignificant.

**Table 8**

| Category | T26 | T26-INS (the primary production) | T26-INS (the secondary production) |
|---|---|---|---|
| EC₅₀ | 0.5081 | 1.223 | 0.9548 |
| Affinity | 1.0 times | 0.4 times | 0.5 times |

### Example 5. Verification of in vivo hair growth effect of in mouse model

In order to verify the hair growth effect of T26 and T26-INS001 of the present disclosure, male C57BL/6 mice (7-week-old) were purchased (BioLink to Korea) and allowed to adapt to the breeding room environment for 1 week. At the age of 8 weeks, the back of the mouse was shaved using a depilator, and the hair was completely removed using a hair removal agent (including 80% thioglycolic acid). Mice having pink skin color with no skin damage at the hair removal site were selectedand randomly divided into test groups and allowed to rest for one day.

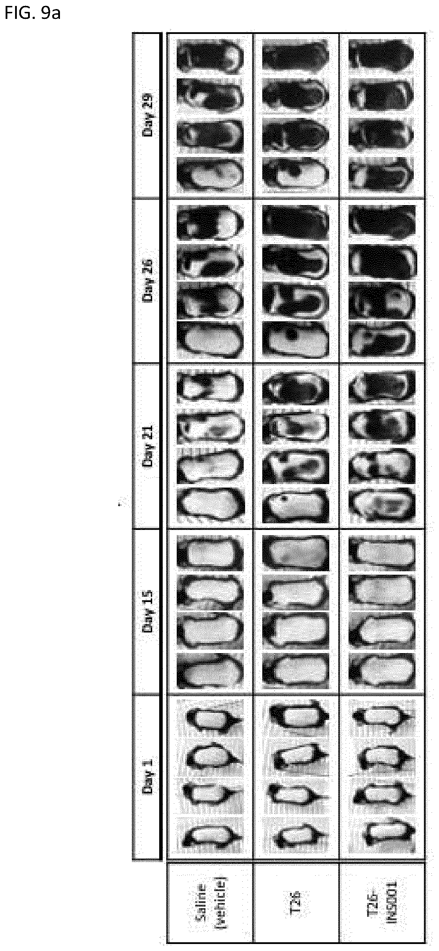

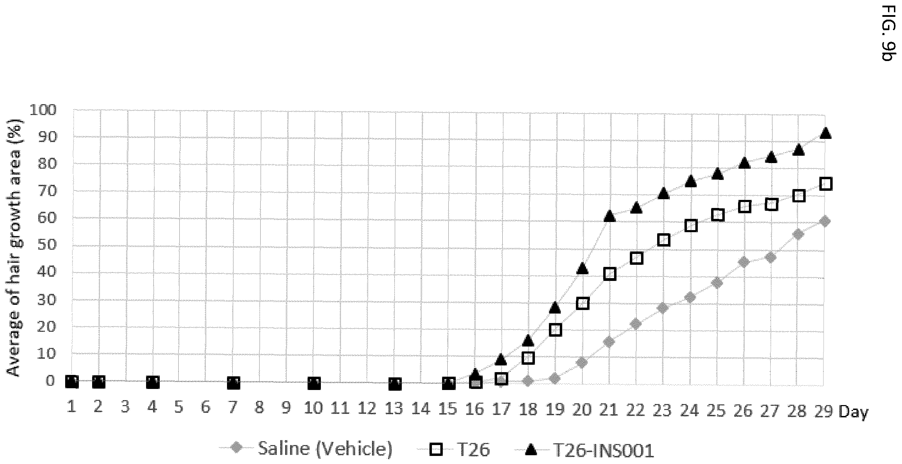

The test group was divided into a negative control group (Saline), a T26 (0.6 µM) administration group, and a T26-INS001 (0.6 µM) administration group with four mice each. A total of 100 µL of the sample was divided into 25 µL and injected intradermally in 4 places, and the frequency of administration was once a day at the same time. The experiment was conducted for a total of 29 days. During the test period, photographs were taken every 3 days, and photographs were taken every day from the 15th day when hair growth began. In order to quantify the degree of hair growth, the area where hair growth starts within the entire hair removing area (the neck area was excluded for reasons of inaccuracy of the numerical value due to skin wrinkles) shown in FIG. 9a was analyzed by an image analysis program (Image J) and quantified as a percentage. The average of 4 mice was graphed (FIG. 9b).

As a result, a clear effect of hair growth was observed in the T26 and T26-INS001 administered groups compared to the negative control group (Saline). Even in numerical data, hair growth was observed in about 60% of the hair removal area in the negative control group at the end of the experiment, but hair growth was observed in 60% in the T26 administration group on day 24, 5 days earlier than the negative control group. In particular, in the T26-INS001 administration group, hair growth was observed at 60% area on day 21, 8 days earlier than the negative control group. At the end of the experiment, all four mice in the T26-INS001 administration group had similar levels of hair as before hair removal.

Although the present invention has been described in detail with reference to the specific features, it will be apparent to those skilled in the art that this description is only for an embodiment and does not limit the scope of the present disclosure. Thus, the substantial scope of the present disclosure will be defined by the appended claims and equivalents thereof.

## Claims

1. A polypeptide that specifically binds to testosterone, comprising an amino acid sequence having at least 94% sequence homology with the following amino acid sequence of SEQ ID NO: 1:

VDNKFNKEMVQAMREISYLPNLNHTQIRAFIWVLFDDPSQSANLLAEAKKL
NDAQAPK (SEQ ID NO: 1).

2. The polypeptide of claim 1, wherein at least one amino acid residue selected from the glutamine residue (Q) at position 11, the serine residue (S) at position 17, and the threonine residue (T) at position 25 of the following amino acid sequence is independently replaced with any amino acid residue:

VDNKFNKEMVQAMREISYLPNLNHTQIRAFIWVLFDDPSQSANLLAEAKKL
NDAQAPK.

3. The polypeptide of claim 1 that specifically binds to testosterone, comprising an amino acid sequence having at least 98% sequence homology with the amino acid sequence of SEQ ID NO: 1.

4. The polypeptide of claim 1, wherein the glutamine residue (Q) at position 11, the serine residue (S) at position 17, or the threonine residue (T) at position 25 of the following amino acid sequence is replaced with alanine residue (A):

VDNKFNKEMVQAMREISYLPNLNHTQIRAFIWVLFDDPSQSANLLAEAKKL
NDAQAPK.

5. The polypeptide of claim 1, wherein the polypeptide comprises an amino acid sequence selected from the group consisting of amino acid sequences of SEQ ID NOs: 5 to 7, 9 to 14, 16, and 20 to 22.

6. The polypeptide of claim 1, wherein the polypeptide comprises all of the underlined amino acid residues in the following amino acid sequence:

VDNKFNKEMVQAMREISYLPNLNHTQIRAFIWVLFDDPSQSANLLAEAKKL
NDAQAPK.

7. A complex comprising a polypeptide that specifically binds to testosterone of claims 1 to 6 and at least one angiogenic polypeptide.

8. The complex of claim 7, wherein the angiogenic polypeptide is osteopontin or a fragment thereof.

9. The complex of claim 8, wherein the fragment of osteopontin comprises the amino acid sequence of SEQ ID NO: 66.

10. A nucleic acid molecule comprising a nucleotide sequence encoding i) the polypeptide of any one of claims 1 to 6, or ii) the complex of claim 7 comprising the polypeptide that specifically binds to testosterone and an angiogenic polypeptide.

11. A recombinant vector comprising the nucleic acid molecule of claim 10.

12. A host cell comprising the recombinant vector of claim 11.

13. A pharmaceutical composition for stimulating hair growth or treating hair loss, comprising: (a) the polypeptide of any one of claims 1 to 6, or the complex of claim 7; and (b) a pharmaceutically acceptable excipient, carrier, or diluent.

14. A cosmetic composition for stimulating hair growth, comprising the polypeptide of any one of claims 1 to 6, or the complex of claim 7.
